# EUROPEAN PATENT SPECIFICATION

(11) **EP 1 919 548 B1**
(45) Date of publication and mention of the grant of the patent: **04.05.2011**
(21) Application number: 06763030.1
(22) Date of filing: 17.08.2006
(51) Int. Cl.: A61M 29/02, A61M 25/10

(54) **CATHETER, IN PARTICULAR PTCA CATHETER**
KATHETER, INSBESONDERE PTCA-KATHETER
CATHÉTER, EN PARTICULIER CATHÉTER POUR ACTP

(30) Priority: 19.08.2005 US 709627 P
(43) Date of publication of application: 14.05.2008
(73) Proprietor: Abbott Laboratories Vascular Enterprises Limited, Dublin 2 (IE)
(72) Inventor: WARNACK, Boris, Mountain View, CA 94040 (US)
(74) Representative: Schmitz, Hans-Werner
(86) International application number: PCT/EP2006/008117
(87) International publication number: WO 2007/020086

(56) References cited:
- US-A- 5 169 386
- US-A- 5 507 766
- US-A1- 2005 171 591

## Description

The invention concerns a catheter, in particular a PTCA catheter, according to the preamble part of claim 1. Such a catheter is known from US 5 169 386 A, US 2005/ 0 171 591 A1 or US 5 507 766 A.

A catheter according to the preamble part of claim 1 usually includes one or two markers, in particular in form of marker bands, made from radiopaque material to identify the position of the balloon within the anatomy when using X-ray imaging methods. Other parts of the catheter, especially the catheter tip, remain nearly invisible for X-ray imaging.

Catheter markers are well known in the art to enable the physician to track the position of the catheter in the patient's body under X-Ray. As described in US5779731 and US5846199 radiopaque markers can be used to indicate the working length of the balloon of a balloon catheter. US5209730 describes a method for accurate placement of a balloon catheter across a stenosis employing cooperating radiopaque markers on the guidewire and the catheter. Further, as depicted in US5669932, markers are employed to indicate the position of the stent in a stent delivery catheter. Markers can be made of diverse materials such as radiopaque metal alloys or polymers doped with radiopaque material as described in US6540721. In special cases, however, it is important for the physician not only to know the accurate position of the balloon but also to know whether the tip of the catheter has already entered a certain region of the lumen the catheter has been introduced in, and in particular whether the catheter has already entered a tight lesion, or not.

It is therefore an object of the present invention to provide a catheter according to the preamble part of claim 1 that is able to facilitate the handling thereof, especially in terms of the identification of the positioning of the catheter tip.

The solution of this object is achieved by the features of claim 1.

The catheter according to the present invention makes it possible to give a tool to the physician to judge whether the tip of the catheter has already entered a certain region of a vessel, especially an occlusion, or not. For the treatment of chronic total occlusion (CTO), the tip and the complete distal part of the catheter has to be very thin and low in diameter, to offer the highest possibility to pass the lesion. As it is not possible to make the tip of a catheter from a radiopaque material or to fill the polymer with a radiopaque agent as this would inevitably thicken the catheter, what would not be acceptable for the reasons given hereinbefore, the catheter of the present application is especially advantageous as it can be kept very thin.

This specific advantage is achieved by the invention and provides that there is a second radiopaque marker, especially proximal of the balloon, that is located proximally at the same distance from the balloon marker neighbouring the tip in the distal direction. This second proximal marker serves as a measuring marker. The physician can thus judge the distance from the first distal marker to the tip by using the known distance between the measuring marker and the first marker that is advantageously located in the balloon.

The markers can be of various materials, such as solid or wired platinum, iridium, gold tantalum etc. The proximal measuring marker should have a different geometry than the first marker (balloon marker) to avoid mixing up and to avoid a balloon dilatation at a wrong position.

The dependent claims contain advantageous embodiments of the present application.

Furthermore, the invention also concerns the use of the catheter according to the present invention in treating vessels, in particular blood vessels, that are blocked especially by a total occlusion. In general, the catheter according to the present invention can be used as a usual PTCA catheter but furnishes the advantage that the physician using the inventive catheter can judge where the catheter tip is located within the vessel by using the known distance between the measuring marker and the balloon marker in order to evaluate where the more or less invisible catheter tip is positioned.

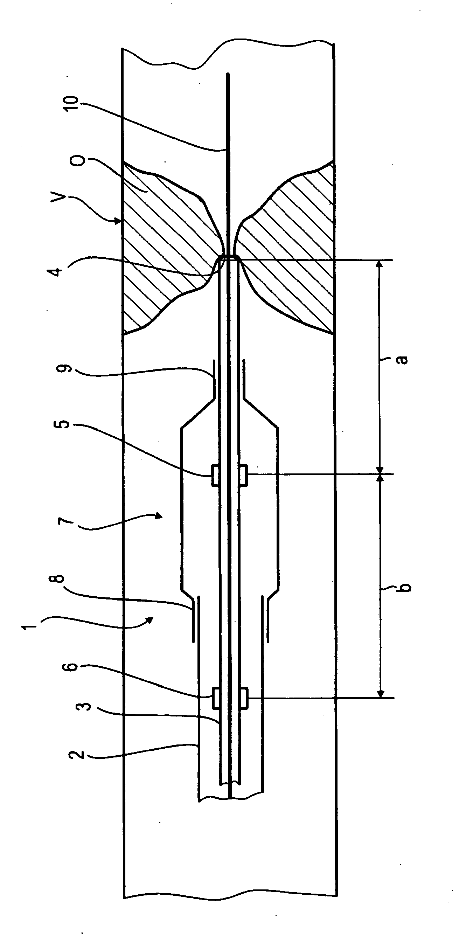

Further features and advantages of the present application will become apparent from the description of the single figure of the drawings.

This single figure is a schematically simplified illustration of a catheter 1, especially in the form of a PTCA catheter. A catheter 1 comprises an outer tube 2 and an inner guide wire tube 3 that is located within the outer tube 2, in particular concentrically oriented with respect to said outer tube 2.

The inner guide wire tube 3 guiding the guide wire 10 includes a tip 4. The single figure of the drawing shows the catheter 1 being located in a vessel V with a tip 4 abutting against a total occlusion O of said vessel V.

For the purpose of visualization of the positioning of the catheter 1 within said vessel V, e.g. by X-ray, the catheter 1 comprises two radiopaque markers 5 and 6 fixed to the inner guide wire tube 3. Marker 5 serves as balloon marker and is centrically positioned in the balloon. The tip 4, however, is virtually invisible. So, according to the principles of the present application, the two markers 5 and 6 are disposed with respect to one another at a known distance b that is set to be equal to a distance a between marker 5 and tip 4.

According to the depicted embodiment, the marker 5 is a first marker being disposed within the balloon 7 that, of course, is not yet inflated in the condition shown in the figure. The marker 6 is disposed proximal of the balloon 7 at said known distance b.

In order to be able to guide the catheter 1 through the occlusion O, it is not possible to dispose another marker at or nearby tip 4 as this would unacceptably thicken the catheter 1.

As, however, the distances a and b are equal or at least nearly equal, the physician can evaluate the positioning of the tip 4 upon visualization of the markers 5 and 6 as he knows that these markers 5 and 6 are disposed with respect to one another at the known distance b.

### Reference Numerals

- 1: Catheter
- 2: Outer tube
- 3: Inner guide wire tube
- 4: Tip
- 5: First marker (balloon marker)
- 6: Second marker (proximal marker)
- 7: Balloon
- 8: Sleeve (fixed to outer tube 2)
- 9: Sleeve (fixed to inner tube 3)
- 10: Guide Wire
- b: known distance between markers 5 and 6
- a: distance between marker 5 and tip 4
- V: Vessel
- O: Occlusion

(The catheter described as a PTCA catheter comprising a balloon can also be a catheter without a balloon according to the principles of the present invention)

## Claims

1. Catheter (1) having a distal portion bearing a functional unit (7) and a catheter tip (4) distal of the functional unit (7),
- the distal portion of the catheter (1) comprising a distal marker (5) and a proximal marker (6),
**characterized in**
- **that** the distance (a) between the distal end of the catheter tip (4) and the center of the distal marker (5) is equal to the distance (b) between the center of the distal marker (5) and the center of the proximal marker (6).

2. Catheter according to claim 1 wherein the functional unit is a balloon (7).

3. Catheter according to claim 1 wherein the distal marker (5) is located in the center of the functional unit (7).

4. Catheter according to claim 1 wherein the distal and the proximal marker (5, 6) are visually distinguishable from each other under x-Ray.

## Patentansprüche

1. Katheter (1) mit einem distalen Bereich, der eine Funktionseinheit (7) trägt, und einer Katheterspitze (4) distal von der Funktionseinheit (7),
- wobei der distale Bereich des Katheters (1) eine distale Markierung (5) und eine proximale Markierung (6) umfasst,
**dadurch gekennzeichnet, dass**
- der Abstand (a) zwischen dem distalen Ende der Katheterspritze (4) und der Mitte der distalen Markierung (5) gleich dem Abstand (b) zwischen der Mitte der distalen Markierung (5) und der Mitte der proximalen Markierung (6) ist.

2. Katheter nach Anspruch 1, wobei die Funktionseinheit ein Ballon (7) ist.

3. Katheter nach Anspruch 1, wobei die distale Markierung (5) in der Mitte der Funktionseinheit (7) liegt.

4. Katheter nach Anspruch 1, wobei die distale Markierung (5) und die proximale Markierung (6) unter Röntgenstrahlung visuell voneinander unterschieden werden können.

## Revendications

1. Cathéter (1) possédant une partie distale supportant une unité fonctionnelle (7) et un embout de cathéter (4) distal de l'unité fonctionnelle (7),
la partie distale du cathéter (1) comprenant un repère distal (5) et un repère proximal (6)
**caractérisé en ce que** la distance (a) entre l'extrémité distale de l'embout de cathéter (4) et le centre du repère distal (5) est égale à la distance (b) séparant le centre du repère distal (5) et le centre du repère proximal (6).

2. Cathéter selon la revendication 1, dans lequel l'unité fonctionnelle est un ballonnet (7).

3. Cathéter selon la revendication 1, dans lequel le repère distal (5) est positionné au centre de l'unité fonctionnelle (7).

4. Cathéter selon la revendication 1, dans lequel les repères distal et proximal (5, 6) peuvent être distingués, de façon visuelle, l'un de l'autre sous rayons X.
